# EUROPÄISCHE PATENTANMELDUNG

(11) **EP 4 506 954 A1**
(43) Veröffentlichungstag der Anmeldung: **12.02.2025**
(21) Anmeldenummer: 23190915.1
(22) Anmeldetag: 10.08.2023
(51) Int. Cl.: G16H 40/40, G16H 40/20, G16H 40/67, G05B 23/02, G16H 20/17

(54) **SYSTEM UND VERFAHREN ZUR ÜBERWACHUNG EINER INFUSIONSPUMPE**

(71) Anmelder: B. Braun Melsungen AG, 34212 Melsungen (DE)
(72) Erfinder: Schwarz, Jan, 34212 Melsungen (DE); Bürger, Maria, 34323 Malsfeld (DE); Angersbach, Klaus, 34326 Morschen (DE)
(74) Vertreter: Tergau & Walkenhorst Intellectual Property GmbH

(57) **Zusammenfassung**

System (2) zur Überwachung einer Infusionspumpe (6), umfassend
• eine Infusionspumpe (6) mit wenigstens einem Sensor (10, 14, 18) zur Erfassung von Sensordaten;
• einen Überwachungs-Server (26) mit einem Überwachungsmodul (26) mit einer Empfangseinheit (36);
• eine Übertragungseinheit (30) zur Übertragung der Sensordaten an die Empfangseinheit (36) des Überwachungsmoduls (26);
wobei das Überwachungsmodul (26) konfiguriert ist, aufgrund der Sensordaten den Zustand der Infusionspumpe (6) zu bestimmen und daraus eine sicherheitsrelevante Klassifizierung der Infusionspumpe (6) abzuleiten.

## Beschreibung

Die Erfindung betrifft ein System und ein Verfahren zur Überwachung einer Infusionspumpe.

Infusionspumpen sind medizinische Geräte, die eine gezielte und automatische Verabreichung von Flüssigkeiten, wie beispielsweise Medikamenten oder Nährstoffen, ermöglichen. Die Sicherheit und korrekte Funktionsweise dieser Pumpen ist von entscheidender Bedeutung, da Fehlfunktionen schwerwiegende Folgen für die Patienten haben können.

Um die Sicherheit der Infusionspumpen zu gewährleisten, werden derzeit verschiedene sicherheitsrelevante Tests durchgeführt. Diese Tests sollen sicherstellen, dass die Pumpen ordnungsgemäß funktionieren, sowohl während der Produktion als auch während des gesamten Lebenszyklus des Geräts. Bekannte technische Sicherheitschecks (TSC) konzentrieren sich auf die Erkennung elektrischer Störungen und mechanischer Defekte der Infusionspumpe und schließen sicherheitsrelevante Arbeiten ein.

Der technische Sicherheitscheck wird von einem speziell dafür geschulten Servicetechniker durchgeführt, um das Gerät zu warten und funktionsfähig zu halten. Der Wartungsprozess ist kompliziert und kann aufwändig sein. Während dieser Tests werden die Infusionspumpen in einen Wartungsmodus versetzt, sodass sie im regulären Krankenhausbetrieb nicht zur Verfügung stehen.

Aus der WO 2021/243068 A1 ist ein System bekannt, welches eine Überwachung von Komponenten über Sensoren in Verbindung mit einem Sever ermöglicht und im Falle einer Fehlfunktion eine Meldung an das betreffende Gerät sendet und eine Wartungsanweisung auf dem Gerät zur Anzeige bringt.

Nachteilig bei bekannten technischen Sicherheitstests ist, dass diese das Außerbetriebnehmen des Infusionspumpe und den Einsatz eines speziellen Technikers erfordern bzw. erst nach einer detektierten Fehlfunktion die Wartung oder Reparatur der Infusionspumpe unterstützen.

Der Erfindung liegt daher die Aufgabe zugrunde, ein System und Verfahren anzugeben, welches die technische Sicherheitskontrollen vereinfacht und automatisiert. Weiterhin sollen ein Computer-Produkt sowie ein Trainingsdatensatz angegeben werden.

In Bezug auf das System wird diese Aufgabe erfindungsgemäß gelöst mit einem System zur Überwachung einer Infusionspumpe mit den Merkmalen von Anspruch 1. Das System umfasst dabei eine Infusionspumpe mit wenigstens einem Sensor zur Erfassung von Sensordaten, einen Überwachungs-Server mit einem Überwachungsmodul mit einer Empfangseinheit und eine Übertragungseinheit zur Übertragung der Sensordaten an die Empfangseinheit des Überwachungsmoduls, wobei das Überwachungsmodul konfiguriert ist, aufgrund der Sensordaten den Zustand der Infusionspumpe zu bestimmen und daraus eine sicherheitsrelevante Klassifizierung der Infusionspumpe abzuleiten.

Vorteilhafte Ausgestaltungen der Erfindung sind Gegenstand der Unteransprüche.

Die Erfindung geht von der Überlegung aus, dass eine Infusionspumpe möglichst ohne Unterbrechungen im medizinischen Betrieb verfügbar sein sollte. Durch die notwendigen technischen Sicherheitsüberwachungen bzw. Sicherheitskontrollen durch einen Servicetechniker, die die Außerbetriebnahme der Pumpe erfordern, steht diese in dieser Zeit nicht zur Verfügung, sodass Ersatzgeräte organisiert werden müssen und organisatorische Maßnahmen ergriffen werden müssen.

Wie nunmehr erkannt wurde, lassen sich technische Sicherheitskontrollen durch einen Servicetechniker durch eine Automatisierung der technischen Sicherheitskontrollen vermeiden, indem der Zustand der Infusionspumpe aufgrund von Sensordaten beurteilt wird. Dies ist insbesondere deshalb möglich, da hierfür Sensoren eingesetzt werden können, welche in der Infusionspumpe bereits vorhanden sind. Sofern diese Daten vorgegebene oder erlernte Bedingungen erfüllen, insbesondere wenn eine Auswahl oder alle Sensordaten innerhalb vorgegebener Toleranzen liegen, kann eine Sicherheitskontrolle als erfüllt gelten, sodass die manuelle Kontrolle entfallen kann und die Infusionspumpe in Betrieb bleiben kann.

Vorteilhafterweise umfasst die sicherheitsrelevante Klassifizierung die Vorhersage eines Ausfalles und/oder über die ordnungsgemäße Funktion der Infusionspumpe über die Laufzeit.

Die sicherheitsrelevante Klassifizierung erfolgt bevorzugt in Zyklen, wobei eine sicherheitsrelevante Überwachung der Infusionspumpe als bestanden gilt, wenn die Infusionspumpe innerhalb des letzten Zyklus ordnungsgemäß funktioniert hat. In diesem Fall kann ein sicherheitsrelevanter manueller Test durch einen Servicetechniker entfallen, sodass die Infusionspumpe weiterhin für ihren Betriebszustand zur Verfügung steht.

Die Übertragungseinheit übermittelt bevorzugt die erfassten Sensordaten im Wesentlichen kontinuierlich oder in regelmäßigen zeitlichen Abständen an die Empfangseinheit. In Abhängigkeit von der Datenlücke werden bevorzugt Interpolationen (linear bzw. Spline) durchführen und die fehlenden Datenwerte approximiert Bei größeren Datenlücken hingegen wird vorteilhafterweise die Trendanalyse abgebrochen und es wird eine Neuinitialisierung durchgeführt. Dies bedeutet im Umkehrschluss, dass die zuvor verwendeten Daten als ungültig erklärt werden und damit die Trendanalyse nicht durchgeführt werden kann.

Das Überwachungsmodul umfasst in einer bevorzugten Ausführungsform ein anpassbares und/oder lernbares Modell zur sicherheitsrelevanten Klassifizierung. Auf diese Weise kann das Modell an die konkrete technische Situation, d.h. die spezifische Pumpe und die spezifischen Sensoren, angepasst werden, und Kriterien für ein ordnungsgemäßes Funktionieren der Infusionspumpe können im Lauf der Zeit an ggf. neu gewonnene Erkenntnisse angepasst werden.

Der Algorithmus des Modells ist vorteilhafterweise ein Prädiktionsansatz, insbesondere umfassend wenigstens ein lineares Filtermodell und/oder ein statistisches Modell, insbesondere umfassend wenigstens ein neuronales Netz und/oder ein Gauß'sches Mixturmodell.

Insbesondere kann das Modell verschiedenen Modelle für unterschiedlichen Gruppen von Sensordaten umfassen. In einer bevorzugten Ausführungsform ist daher eine Mehrzahl von Modellen vorgesehen, wobei jedes Modell spezifisch für eine Auswahl der gesamten Sensordaten eingesetzt wird. So können bestimmte Sensordaten binär sein, wobei ein Sensordatenwert unmittelbar zur Klassifizierung des Ausfalls oder der Fehlfunktion führt. Andere Sensordaten bzw. ihre Kombinationen können zu komplexeren Klassifikationen mit Zwischenstufen führen.

Für jeden Sensor existieren vorteilhafterweise unterschiedliche Modelle, die zunächst einmal nicht korreliert sind. Die Korrelation erfolgt lediglich dadurch, dass im Falle eines Ausfalls, d.h. Defekts die Infusionspumpe generell als defekt markiert wird bzw. im Falle eines "behebbaren" Fehler, wie der Displaykalibrierung, eine Kalibrierung durch den Nutzer gestartet/initiiert wird.

Das Überwachungsmodul ist vorzugsweise konfiguriert, in einer Anpassungs- und/oder Lernphase die Parameter des Modells einzustellen. Das heißt, die Parameter des Modells werden mit Hilfe eines Trainingsdatensatzes festgelegt. Das Training kann in Zyklen erfolgen. Vorteilhafterweise wird bei einem neuronalen Netz das Netz durch überwachtes Lernen mit Hilfe des Trainingsdatensatzes trainiert. In einer bevorzugten Ausführungsform wird der Lernerfolg des neuronalen Netzes dann mit Hilfe eines Testdatensatzes geprüft. Sofern vorgegebene Testkriterien nicht erfüllt sind, kann das neuronale Netz mit veränderten Trainingsparametern (beispielsweise Lernrate) und/oder verändertem Trainingsdatensatz trainiert werden. Analog ist dies auch für auf das Gauß'sche Mixturmodell anwendbar. Auch hier wird der Lernerfolg mit anderen Daten überprüft.

Bei einer Spritzenpumpe erfasst und analysiert (d.h. wertet mit Hilfe des Modells aus) das Überwachungsmodul bevorzugt Sensordaten aus der Gruppe:
- Krallenwinkel, korreliert mit der verwendeten Spritze und ihrer Länge / ihrem Durchmesser als Funktion Zeit;
- Spritzenlänge / -durchmesser der verwendeten Spritze als Funktion der Zeit;
- Druckwerte während des Einlegens der Spritze und/oder während der Infusion.

Bei einer Volumenpumpe/Peristaltikpumpe erfasst und analysiert (d.h. wertet mit Hilfe des Modells aus) das Überwachungsmodul bevorzugt Sensordaten aus der Gruppe:
- Bewegung der Türpositionssensoren durch Überprüfung der Widerstandswerte über die Zeit und Korrelation der Werte mit den Motorschritten;
- Farbdetektion des eingelegten Schlauches, insbesondere anhand seiner Klemme, wobei der gemessene Farbwert mit der Bestätigung/Änderung durch den Benutzer ausgewertet wird;
- Erfassung von Drucksensorwerten über die Zeit, um zu überprüfen, ob sich im Laufe der Lebensdauer Abweichungen/Änderungen ergeben.

Bei einer Volumenpumpe/Peristaltikpumpe mit einem Display oder Spritzenpumpe mit einem Display erfasst und analysiert (d.h. wertet mit Hilfe des Modells aus) das Überwachungsmodul bevorzugt Sensordaten aus der Gruppe:
- Berührungssensorwerte an definierten Positionen auf dem Display, z.B. Schaltfläche "Start der Infusion" oder in Eingabefeldern als Funktion der Zeit;
- Austausch von Batterieinformationen durch ein Gerätemodell, wie beispielsweise der Ladezustand als Funktion der Zeit, die Kapazität des Akkus, die Anzahl der Ladezyklen.

Das System umfasst bevorzugt eine Transmissionseinrichtung zur Übertragung von Informationen von dem Überwachungsmodul an die Infusionspumpe. Vorteilhafterweise sind die Empfangseinheit und die Transmissionseinrichtung integriert bzw. kombiniert gebildet, d.h. die gleiche Einheit empfängt und sendet.

Das Überwachungsmodul ist vorzugsweise ausgebildet zu überwachen, ob die Sensordaten wenigstens eines Sensors in einem vorgegebenen und/oder gelernten Nominalbereich liegen, wobei das Überwachungsmodul ausgebildet ist mit Hilfe der Transmissionseinrichtung an die Infusionspumpe Instruktionen zu senden und/oder ein Wartungsprogramm, bevorzugt eine Displaykalibrierung, der Infusionspumpe zu initiieren. Dies erfolgt bevorzugt bei Sensordaten zu Eigenschaften der Infusionspumpe, welche sich in einem Wartungsschritt durch medizinisches Fachpersonal, insbesondere kurzfristig, ändern lassen. Beispielsweise kann eine Neu-Kalibirierung des Touch-Screens bzw. Touch-Displays erfolgen.

Die Instruktionen und/oder Schritte des Wartungsprogramm werden bevorzugt auf einem Display der Infusionspumpe angezeigt. Zusätzlich oder alternativ können auch akustische Signale oder Sprachnachrichten verwendet werden.

Die Übertragungseinheit und die Empfangseinheit sind bevorzugt zu drahtgebundener oder drahtloser Kommunikation, insbesondere mit Funk, Mobilfunk, W-Lan, Bluetooth ausgebildet. Auf diese Weise müssen keine zusätzlichen Kabel oder Leitungen verlegt werden, was den Arbeitsbereich der Infusionspumpe übersichtlicher gestaltet.

Das System umfasst vorteilhafterweise weiterhin einen Gesundheitsdaten-Server, welcher zum Empfang von Daten von dem Überwachungsmodul und/oder zur Übertragung von Daten an das Überwachungsmodul ausgebildet ist. Der Server empfängt die Daten bevorzugt kontinuierlich, d.h. immer dann wenn sich eine Änderung ergibt. Bei beispielsweise laufender Therapie/Förderung werden kontinuierlich Drucksensorwerte übermittelt und überwacht.

Beim Einlegen einer Spitze in die Spritzenpumpe wird jedoch primär während des Einlegevorgangs der Krallenwinkel sowie deren Durchmesser gemessen. Danach erfolgt die Messung und das Reporting in unregelmäßigen Abständen (z.B. in 50 ms bis 5 s Abständen), jedoch immer dann, wenn eine neue Spritze eingelegt wird. Dasselbe gilt für den Luftsensor der Peristaltikpumpe. Hierbei werden die Daten kontinuierlich bei eingelegtem Einmalartikel gemessen. Die Daten werden, wenn es möglich ist, gebündelt an den Server übergeben. Dies geschieht einerseits Event-gesteuert, z.B. wenn sich eine Flussrate ändert, eine Spitze/Schlauch eingelegt oder gewechselt wird, und in jedem Fall bevorzugt alle 5 s.

Der Gesundheitsdaten-Server ist vorteilhafterweise als Cloud-Server ausgebildet.

Der Überwachungs-Server umfasst vorteilhafterweise ein Trendanalysemodul, welches dazu ausgebildet ist, aufgrund übermittelter Sensordaten eine Trendanalyse durchzuführen. Das Trendanalysemodul ist bevorzugt software- und/oder hardwaremäßig im Überwachungs-Server ausgebildet.

Aufgrund der Trendanalyse führt das Trendanalysemodul vorzugsweise eine Lebenszeiteinschätzung der Infusionspumpe durch. Durch die Trendanalyse können vorzeitig Fehler erkannt und auf diese reagiert werden. Bevorzugt wird eine vorsorgliche Wartung dem Techniker mitgeteilt und ggf. eine Nachricht an die Pumpe gesendet, sie zum Service zu geben. Letzteres hängt jedoch davon ab, ob die Pumpe noch an einem Patienten betrieben wird oder ob sie ungenutzt ist. Im letzteren Fall ist denkbar, die Pumpe mit einem Service-Hinweis zu versehen. Im Falle eines Touch-Displays erfolgt in einer bevorzugten Ausführungsform das Senden eines "Bitte neu kalibrieren"-Feldes denkbar, dass durch die Pflegerin entsprechend bedient wird. Dies erfolgt nur außerhalb der laufenden Therapie. Hier kann insbesondere durch schleichende Prädiktion früh erkannt werden, dass sich ein Fehler im Lauf der Zeit verschlimmert oder die erwartete Laufzeit/Lebenszeit der Infusionspumpe angepasst werden.

Als Beispiel kann in einem Trend erkannt werden, dass das Touch-Display eine Neukalibrierung benötigt. Dies ist als Trend beispielsweise daran erkennbar, dass die Pixelpositionen, die den Touch-Gesten des Anwenders entsprechen, für eine Schaltfläche, deren Position bekannt ist, sich stetig verändern. Der Anwender muss beispielsweise immer weiter oben auf das Display drücken, um die Schaltfläche zu aktivieren.

In Bezug auf das Verfahren wird die oben genannte Aufgabe erfindungsgemäß gelöst durch ein Verfahren mit den Schritten: Erfassen von Sensordaten einer Infusionspumpe; Eingeben der Sensordaten in ein mathematisches Modell; Klassifizieren des Zustandes der Infusionspumpe durch das mathematische Modell. Das Verfahren ist computerimplementiert.

In einer bevorzugten Ausführungsform umfasst das Verfahren einen Anpassungs- und/oder Trainingsschritt für das mathematische Modell, in welchem mit Hilfe eines Trainingsdatensatzes das Modell angepasst und/oder trainiert wird.

Das Trainingsverfahren der statistischen Modelle enthält bevorzugt nur Sensordaten, die sowohl zu einem Ausfall geführt haben bzw. zu einem fehlerfreien Zustand gehören. Es ist a-priori bekannt, ob es ein Gutfall (Fehlerfreiheit) oder Schlechtfall (Ausfall) ist. Die mathematischen/statistischen Modelle werden entsprechend darauf trainiert. Es kann auch eine Adaptionsphase vorgesehen sein, in der die statistischen Modelle durch weitere Daten (Messwerte) aus dem Feld angepasst und verbessert werden. Dies ist vorteilhaft, wenn die Sensordaten einer höheren Varianz ausgesetzt sind. Diese Varianz lässt sich durch ein Nachtrainieren, also eine Adaption, in das gelernte Modell einpflegen und dadurch zu einer größeren Robustheit führen. Es ist noch eine Adaption an die Realität möglich, d.h. das Modell wird mit Realdaten immer weiter verbessert/adaptiert.

Der Trainingsdatensatz umfasst vorteilhafterweise Daten wenigstens eines Sensors und eine zugeordnete Klassifizierung. Vorteilhafterweise fließt mehr als ein Sensor in die Betrachtungen ein. Die Sensorwerte sind unkorreliert und daher separat zu betrachten. Bei den Trainingsdaten sind Gutfälle und Schlechtfälle erforderlich, um eine Klassifizierung zu ermöglichen

Das mathematische Modell umfasst vorteilhafterweise wenigstens ein Modell aus der Gruppe: lineares Filtermodell und/oder ein statistisches Modell, insbesondere umfassend wenigstens ein neuronales Netz und/oder ein Gauß'sches Mixturmodell.

Das Verfahren umfasst bevorzugt einen Verfahrensschritt des Überwachens der Sensordaten wenigstens eines Sensors, wobei auf einem Display der Infusionspumpe Wartungsinstruktionen angezeigt werden, sofern die Sensordaten einen vorgegebenen und/oder gelernten Nominalbereich verlassen.

Wartungsinstruktionen bezeichnen hierbei im Wesentlichen Instruktionen zur Neukalibrierung des Displays bzw. Touchkalibrierung. Bei einem anderem Defekt kann die entsprechende Wartungsinstruktion ein Hinweis sein, dass die Pumpe an den Servicetechniker zu übergeben ist (weil ein Defekt vorliegt).

In Bezug auf das Computer-Produkt wird die oben genannte Aufgabe erfindungsgemäß gelöst durch ein Computerprogramm-Produkt umfassend Instruktionen, welche, wenn sie auf einem Computer ausgeführt werden, ein oben beschriebenes Verfahren durchführen.

In Bezug auf den Trainingsdatensatz zum Trainieren eines neuronalen Netzes in einem Verfahren mit einem oben beschriebenen Trainingsschritt wird die oben genannte Aufgabe erfindungsgemäß gelöst mit einem Trainingsdatensatz, welcher die folgende Struktur aufweist. Der Trainingsdatensatz enthält bevorzugt nur Sensordaten, die sowohl zu einem Ausfall geführt haben bzw. zu einem fehlerfreien Zustand gehören. Es ist a-priori bekannt, ob es ein Gutfall (Fehlerfreiheit) oder Schlechtfall (Ausfall) ist. Die mathematischen/statistischen Modelle werden entsprechend darauf trainiert.

In einer Adaptionsphase können die statistischen Modelle durch weitere Daten (Messwerte) aus dem Feld angepasst und verbessert werden. Dies ist insbesondere dann vorteilhaft, wenn die Sensordaten einer höheren Varianz ausgesetzt sind. Diese Varianz lässt sich durch ein Nachtrainieren, also eine Adaption, in das gelernte Modell einpflegen und dadurch zu einer größeren Robustheit führen. Der Trainingsdatensatz umfasst gewissermaßen das a-priori Wissen über defekte und fehlerfreie Sensordaten. Zudem werden bevorzugt die Sensordaten einer unterschiedlichen Anzahl an Infusionspumpen (Schlauch- bzw. Spritzenpumpen) für das Training herangezogen, um die Streuung der Sensoren durch Bauteiltoleranzen abzudecken. Ferner werden vorteilhafterweise die Sensordaten von Infusionspumpen aus unterschiedlichen Chargen und eines unterschiedlichen Alters verwendet, um damit die Streuung der Sensordaten besser/genauer abzubilden.

Die Erfindung betrifft auch ein System zur Überwachung einer ambulatorischen Pumpe oder einer Dialysemaschine, umfassend eine ambulatorische Pumpe bzw. Dialysemaschine mit wenigstens einem Sensor zur Erfassung von Sensordaten, einen Überwachungs-Server mit einem Überwachungsmodul mit einer Empfangseinheit, eine Übertragungseinheit zur Übertragung der Sensordaten an die Empfangseinheit des Überwachungsmoduls, wobei das Überwachungsmodul konfiguriert ist, aufgrund der Sensordaten den Zustand der ambulatorischen Pumpe / Dialysemaschine zu bestimmen und daraus eine sicherheitsrelevante Klassifizierung der ambulatorischen Pumpe / Dialysemaschine abzuleiten. Die oben für das System zur Überwachung einer Infusionspumpe in den Ansprüchen oder der Beschreibung beschriebenen bevorzugten Ausführungsformen sind auch für dieses System zur Überwachung einer ambulatorischen Pumpe oder einer Dialysemaschine bevorzugte Ausführungsformen, sofern sie technisch direkt oder äquivalent für den Fachmann übertragbar sind.

Die Vorteile der Erfindung liegen insbesondere darin, dass ein Automatismus bereitgestellt wird, um Ausfälle frühzeitig zu erkennen und/oder die Wartung des Gerätes aufgrund von Trendanalysen gestützt auf Sensorwerten zu verlängern.

Auf diese Weise kann die Infusionspumpe möglichst lange in Betrieb bleiben. Durch die Überwachung der Infusionspumpe und/oder die durchgeführten Trendanalysen können Wartungsintervalle nach hinten geschoben oder vorverlegt werden, wodurch der funktionsfähige Einsatz der Infusionspumpe sichergestellt werden kann. Da generell eine Wartung alle zwei Jahre im deutschen Raum durchgeführt werden muss, wird durch die kontinuierliche Überwachung ermöglicht, die Prüfung aufgrund der Datenlage durchzuführen. Damit werden Zeit und entsprechend Geld gespart.

Ein Ausführungsbeispiel der Erfindung wird anhand einer Zeichnung näher erläutert. Darin zeigen in stark schematisierter Darstellung:
- FIG. 1: ein System in einer bevorzugten Ausführungsform mit einer Infusionspumpe, einem Überwachungs-Server und einem Gesundheitsdaten-Server;
- FIG. 2: ein System in einer weiteren bevorzugten Ausführungsform mit einer Infusionspumpe, einem Überwachungs-Server und einem Gesundheitsdaten-Server;
- FIG. 3: ein Display einer Infusionspumpe in einem ersten Zustand;
- FIG. 4: das Display gemäß FIG. 3 in einem zweiten Zustand;

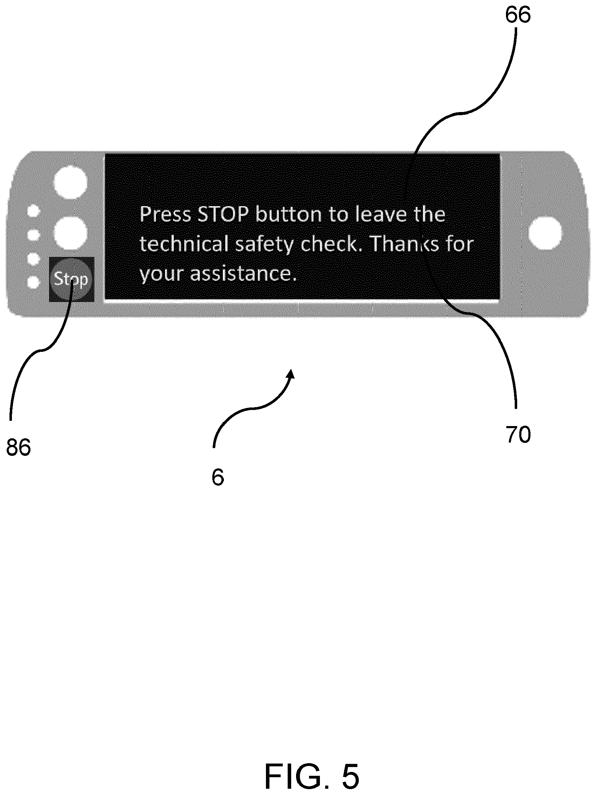

- FIG. 5: das Display gemäß FIG. 3 in einem dritten Zustand, und
- FIG. 6: ein Ablaufdiagramm eines Verfahrens in einer bevorzugten Ausführung.

Gleiche Teile sind in allen Figuren mit denselben Bezugszeichen versehen.

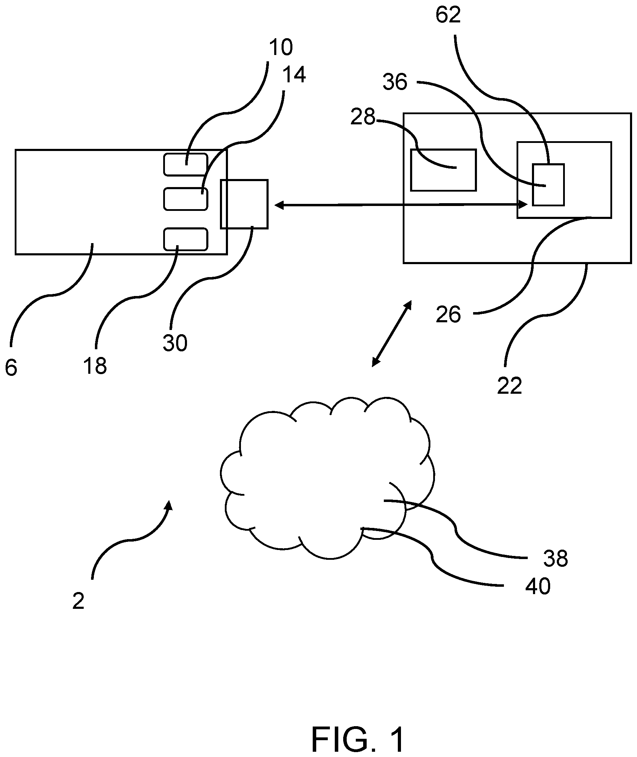

Ein in FIG. 1 dargestelltes System 2 umfasst eine Infusionspumpe 6 mit einer Mehrzahl von Sensoren 10, 14, 18. Das System 2 umfasst einen Überwachungs-Server 22 mit einem Überwachungsmodul 26, welches hardware- und/oder softwaremäßig in dem Überwachungs-Server 22 implementiert ist. Das System 2 umfasst weiterhin eine Übertragungseinheit 30, welche mit den Sensoren 10, 14, 18 oder einen Prozessor, mit welchem die Sensoren verbunden sind, verbunden ist und welche konfiguriert bzw. eingerichtet ist, die von den Sensoren erfassten Daten bzw. Sensordaten an eine Empfangseinheit 36 des Überwachungsmoduls 26 zu übermitteln.

Das System 2 umfasst weiterhin einen Gesundheitsdaten-Server 38, der vorliegend bevorzugt als Cloud-Server 40 ausgebildet, wobei der Überwachungs-Server 22 und der Cloud-Server 40 für eine Datenübertragung von dem Überwachungs-Server 22 zum Cloud-Server und/oder vom Cloud-Server 40 zum Überwachungs-Server 22 eingerichtet sind.

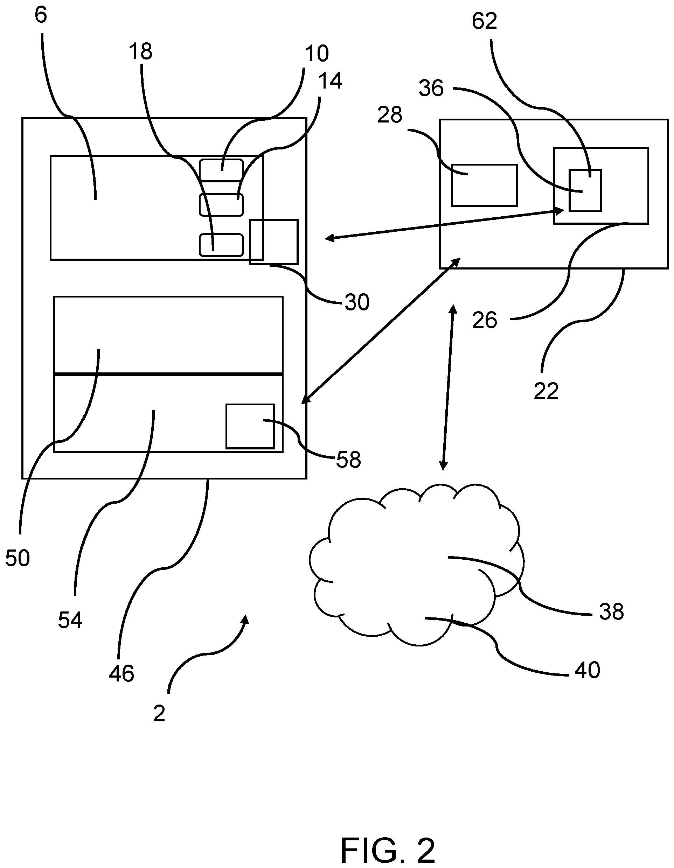

In FIG. 2 ist ein System 2 in einer weiteren bevorzugten Ausführungsform dargestellt. Das System 2 umfasst einen Infusionsarbeitsplatz 46, welcher die Infusionspumpe 6 sowie ein Rack-System 50 und ein Kommunikationssystem 54 umfasst. In dieser Ausführung weist der Infusionsarbeitsplatz 46 eine Übertragungseinheit 30 auf. Das Kommunikationssystem 54 kann eine Übertragungseinheit 58 zum Datenaustausch mit dem Überwachungs-Server 22 aufweisen. Der Infusionsarbeitsplatz 46 ist dazu ausgebildet, mehrere Infusionspumpen 6, insbesondere bis zu 24 Infusionspumpen, in dem Rack-System 50 aufzunehmen, welches ein Aufnahmesystem für die Infusionspumpen 6 bildet. In dieser Ausbildung ist somit eine gemeinsame Übertragungseinheit 30 für alle im Rack-System 50 angeordneten Infusionspumpen 6 vorgesehen sein. Jede Pumpe sendet ihre Daten bevorzugt einzeln über ihre Kommunikationseinheit an den Server 22. Jede Pumpe besitzt eine eindeutige Identifikation, wodurch sie entsprechend zugeordnet werden kann.

Die beiden in den FIG. 1 und 2 dargestellten Systeme 2 weisen jeweils eine Transmissionseinrichtung 62 zur Übertragung von Informationen von dem Überwachungsmodul 26 an die Infusionspumpe 6 auf. Vorliegend sind Empfangseinrichtung 36 und Transmissionseinrichtung 62 integriert ausgeführt.

Der Überwachungs-Server 22 der Systeme 2 umfasst weiterhin jeweils ein Trendanalysenmodul 28.

Die Sensoren 10, 14, 18 können Sensoren sein aus der Gruppe:
- Sensor zur Messung des Krallenwinkels
- Sensor zur Messung des Spritzendurchmessers
- Drucksensor zur Messung des Drucks in der Spritze
- Türsensor einer Volumenpumpe bzw. Peristaltikpumpe
- Farbdetektionssensor für eine Peristaltikpumpe
- Touch-Display-Sensor
- Sensor für Batterieladezustand
- Geräteseitige Klemme der Peristaltikpumpe (zur Überwachung, ob der Schlauch ggf. abgeklemmt ist, z.B. wenn die Tür geöffnet wird)
- Sensor zur Überprüfung einer eingesteckten Schlauchklemme bei der Peristaltikpumpe
- Verbindung mit Netzspannung (ja/nein)
- Stellung des Kolbenmessers bei einer Spritzenpumpe

Vorzugsweise wird der Schwesternruf überwacht, d.h. es wird überwacht, ob das Signal tatsächlich am Konnektor anliegt. Vorzugsweise erfolgt eine Überwachung der LEDs (rot, gelb, weiß). Hierzu werden die Spannungswerte auf Plausibilität/Drift prüfen und entsprechend einem Modell zugeführt.

Das Überwachungsmodul 26 in der in den in FIG. 1 und 2 dargestellten Ausführungsformen ist ausgebildet, aufgrund der eingehenden Sensordaten der Sensoren 10, 14, 18 eine Klassifizierung des Zustands der Infusionspumpe 6 durchzuführen. Das Überwachungsmodul 26 ist dazu ausgebildet, angepasst bzw. trainiert zu werden, um die genannte Klassifizierung des Zustands durchzuführen. In Ausführungen, in denen das Modell ein statistisches Modell, insbesondere neuronales Netz ist, wird ein Trainingsdatensatz bereitgestellt, welcher Sensordaten enthält. Weiterhin wird ein Testdatensatz bereitgestellt, um die Qualität bzw. Genauigkeit des trainierten Modells zu testen.

Bevorzugt wird hierbei Supervised Learning durchgeführt, um sicher zu sein, dass das Modell korrekt trainiert wird. Reinforced Learning kann auch eingesetzt werden unter der Voraussetzung, dass die zugrundeliegende Markov-Kette verständlich/nachvollziehbar ist, d.h., dass das Markov-Modell bzgl. der Gegebenheiten validiert ist. Ein beispielhafter Trainingsdatensatz enthält die Messwerte zur Spritzengrößenerkennung. Beim Einlegen der Spritze in eine Spritzenpumpe wird der Durchmesser anhand von Widerstandswerten gemessen. Diese Widerstandwerte variieren einerseits in Abhängigkeit der Spritzengröße (z.B. hat eine 50 ml Spritze einen größeren Durchmesser als eine 20 ml Spritze) und andererseits in Abhängigkeit von der Einlage. Es ist überdies bekannt, wie der Nominaldurchmesser der Spitze ist und ob er sich um ein Gutmuster oder Schlechtmuster handelt.

Ein ähnliches Beispiel lässt sich für die Position der Türstellung ermitteln. Ein beispielhaftes System besitzt insgesamt 3 unterschiedliche Positionen der Türstellung (auf, zu und "Parkposition"). Aufgrund von Bauteiltoleranzen variieren diese Messwerte ebenfalls um einen Nominalwert für diese Positionen. Auch hier lassen sich durch Schwellwerte Gut- und Schlechtmuster erzeugen, die für das Training verwendet werden.

Weiterhin lassen sich die Touchpositionen auf dem Display bzw. Bildschirm relativ leicht als Eingangsgröße wählen. Der Trainingsdatensatz kennt die Positionen zu einzelnen grafischen Elementen auf dem Bildschirm sowie eine Varianz der Datenpunkte zu einem Nominalwert. Liegt der Messwert innerhalb dieses Bereiches, wird er als gut angesehen. Liegt ein Messwert außerhalb eines Bereiches, dass ist es als falsch markiert.

Eine Hauptaufgabe des aktuellen technischen Sicherheitschecks besteht darin, zu prüfen, ob Tasten auf Berührungen reagieren bzw. ob die Alarmanzeigen der Infusionspumpe (z. B. gelbes Statuslicht, rotes Statuslicht, grünes Infusionsstatuslicht) ordnungsgemäß funktionieren. Aktuell müssen Geräte alle 2 Jahre einer Sicherheitskontrolle unterzogen werden. Die vorliegende Erfindung ermöglicht, dass einzelne Prüfungen an das Fachpersonal ausgelagert werden (Beispiel LEDs checken oder Touchscreen testen), um damit Geld und Zeit zu sparen. Ein zweiter Aspekt der Erfindung bezieht sich darauf, dass aufgrund einer laufenden Überwachung des Gerätes und seiner Messwerte, sehr deutlich erkannt werden kann, ob sich ein Fehler einschleicht oder eben auch nicht. Damit besteht die Möglichkeit, diesen Anteil zu automatisieren und damit die Notwendigkeit der dedizierten Sicherheitskontrolle zu umgehen, weil die Daten laufend (oder in kürzeren Intervalle als es eine Sicherheitskontrolle vorsehen würde) überprüft werden.

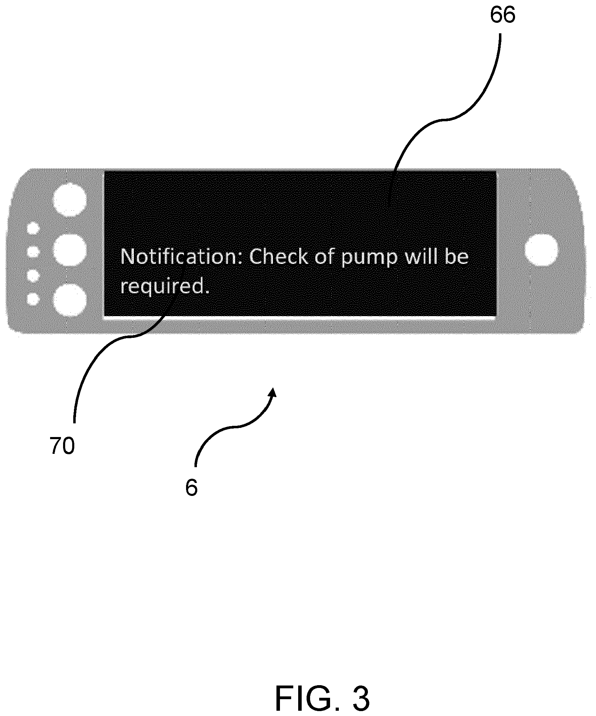

Ein Aspekt der vorliegenden Erfindung ist daher, durch den Überwachungs-Server 22 Informationsnachrichten an die Infusionspumpe 6 zu schicken und dadurch den Anwender bzw. das medizinische Fachpersonal zu informieren. Vorliegend wird, wie in FIG. 3 dargestellt, das medizinische Personal dahingehend durch eine Nachricht 70 auf einem Display 66 der Infusionspumpe 6, das als Touch-Display ausgebildet ist, informiert, dass ab dem nächsten Tag bei den nicht infundierenden Pumpen eine Meldung zur Überprüfung der Statusleuchten angezeigt wird. Diese Meldung wird nur angezeigt, wenn die Infusionspumpe inaktiv ist.

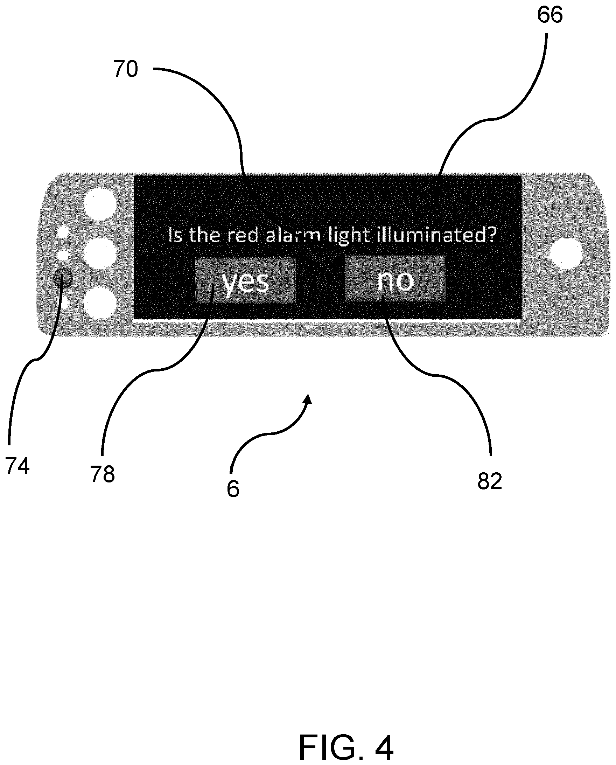

Unabhängig davon, ob eine solche Nachricht gepostet wird oder nicht, wird der Überwachungs-Server 22 auf dem Display 66 der Infusionspumpe 6 eine Nachricht 70 anzeigen, dass der Anwender die Statuslichter überprüfen soll, wie dies in FIG. 4 beispielhaft für ein rotes Alarmlicht 74 dargestellt ist.

Die Statuslichter werden der Reihe nach aktiviert und der Anwender muss bestätigen, dass das jeweilige Statuslicht geleuchtet hat. Auf dem Touchbildschirm wird eine Bestätigung durchgeführt, um auch die Reaktion des Displays 66 auf Touch-Eingaben zu überprüfen. Dazu werden auf dem Display zwei Schaltflächen 78, 82 eingeblendet, wobei eine Schaltfläche 78 die Antwort "Ja" und die andere Schaltfläche die Antwort "Nein" repräsentiert.

Nachdem des jeweilige Statuslicht angezeigt und bestätigt wurde, erhält der Anwender eine kurze Nachricht, um eine Hardkey-Taste (also keine Taste auf dem Touch-Display, sondern eine mechanische Taste) zu drücken, um den Dialog zu verlassen. Durch die Verwendung von Touchscreen und Hardkey-Tasten zur Bestätigung der Instruktionen werden auch die Pflichtfunktionalitäten der Nutzer-Schnittstelle überprüft. Ein oben beschriebener Dialog mit dem Anwender durch dargestellte Instruktionen und Betätigung von Tastenkann kann auch durch akustische Signale zur Überprüfung des Lautsprechers und/oder eines Backup-Alarms ergänzt werden.

Schließlich wird nach Abschluss aller Tests der technische Sicherheitscheck beendet, und dem Anwender wird auf dem Display 66 eine entsprechende Nachricht 70 gezeigt, die er durch Drücken auf eine Stop-Taste 86, welche vorliegend als Hardkey-Taste ausgebildet ist, quittieren kann, wodurch der Sicherheitscheck beendet wird.

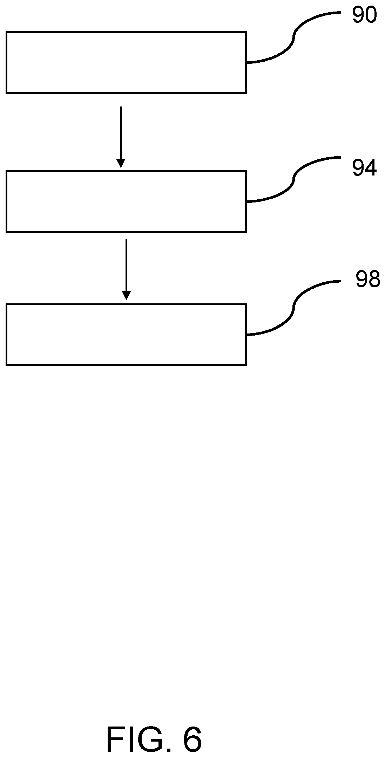

In FIG. 6 ist ein Verfahren zur Überwachung einer Infusionspumpe 6 in einer bevorzugten Ausführungsform dargestellt. In einem ersten Verfahrensschritt 90 werden Sensordaten einer Infusionspumpe 6 erfasst, welche von Sensoren gemessen werden. Die Erfassung der Sensordaten erfolgt dabei kontinuierlich oder in regelmäßigen Abständen. In einem zweiten Schritt 94, der zumindest teilweise parallel zu dem ersten Schritt 90 durchgeführt wird, werden die Sensordaten in ein mathematisches Modell eingegeben, dies erfolgt automatisiert und in regelmäßigen Abständen. Das Modell ist ein lineares Filtermodell und/oder ein statistisches Modell, insbesondere umfassend wenigstens ein neuronales Netz und/oder ein Gaußsches Mixturmodell.

In einem dritten Verfahrensschritt 98, welcher zumindest teilweise parallel zu Verfahrensschritt 94 abläuft, wird der Zustand, insbesondere der sicherheitsrelevante Zustand, der Infusionspumpe 6 klassifiziert. Die spezifische Klassifizierung kann je nach Modell unterschiedlich ausgestaltet sein. So kann bei einfachen linearen Modellen die Klassifizierung eine binäre Entscheidung darstellen: Zustand ist aus sicherheitstechnischen Aspekten OK / Zustand ist nicht OK; die Infusionspumpe muss gewartet bzw. repariert werden.

Die Sensordaten werden gemessen und gegenüber dem Modell abgeglichen. Dies bedeutet, dass ausgehend vom Sensorwert und den zuvor gemessenen Werten eine Einschätzung/Prädiktion (über Filterverfahren oder maschinelles Lernen) eines Zustandes "Gerät defekt", "Gerät betriebsbereit" erhält.

Wird das Gerät als "Gerät betriebsbereit" markiert, so geschieht gar nichts. Das Gerät kann weiterhin verwendet werden. Wird jedoch der Zustand "Gerät defekt" geschätzt, so wird dieses in Form einer Nachricht auf dem Gerät angezeigt (in Form einer Fehlermeldung). Zusätzlich wird auf dem Server eine Nachricht an den Servicetechniker erstellt. Es ist auch möglich, E-Mails oder sonstige Nachrichten über den Server an den Servicetechniker zwecks Instandsetzung der Infusionspumpe zu senden.

### Bezugszeichenliste

- 2: System
- 6: Infusionspumpe
- 10: Sensor
- 14: Sensor
- 18: Sensor
- 22: Überwachungs-Server
- 26: Überwachungsmodul
- 28: Trendanalysenmodul
- 30: Übertragungseinheit
- 36: Empfangseinheit
- 38: Gesundheitsdaten-Server
- 40: Cloud-Server
- 46: Infusionsarbeitsplatz
- 50: Rack-System
- 54: Kommunikationssystem
- 58: Übertragungseinheit
- 62: Transmissionseinrichtung
- 66: Display
- 70: Nachricht
- 74: rotes Alarm licht
- 78: Schaltfläche
- 82: Schaltfläche
- 86: Stop-Taste
- 90: Schritt
- 94: Schritt
- 98: Schritt

## Patentansprüche

1. System (2) zur Überwachung einer Infusionspumpe (6), umfassend
• eine Infusionspumpe (6) mit wenigstens einem Sensor (10, 14, 18) zur Erfassung von Sensordaten;
• einen Überwachungs-Server (26) mit einem Überwachungsmodul (26) mit einer Empfangseinheit (36);
• eine Übertragungseinheit (30) zur Übertragung der Sensordaten an die Empfangseinheit (36) des Überwachungsmoduls (26);
wobei das Überwachungsmodul (26) konfiguriert ist, aufgrund der Sensordaten den Zustand der Infusionspumpe (6) zu bestimmen und daraus eine sicherheitsrelevante Klassifizierung der Infusionspumpe (6) abzuleiten.

2. System (2) nach Anspruch 1, wobei die sicherheitsrelevante Klassifizierung die Vorhersage eines Ausfalles und/oder über die ordnungsgemäße Funktion der Infusionspumpe (6) über die Laufzeit umfasst.

3. System (2) nach Anspruch 2, wobei die sicherheitsrelevante Klassifizierung in Zyklen erfolgt, und wobei eine sicherheitsrelevante Überwachung der Infusionspumpe (6) als bestanden gilt, wenn die Infusionspumpe (6) innerhalb des letzten Zyklus ordnungsgemäß funktioniert hat.

4. System (2) nach einem der Ansprüche 1 bis 3, wobei die Übertragungseinheit (30) die erfassten Sensordaten im Wesentlichen kontinuierlich oder in regelmäßigen zeitlichen Abständen an die Empfangseinheit (36) übermittelt und/oder
wobei das Überwachungsmodul (26) ein anpassbares und/oder lernbares Modell zur sicherheitsrelevanten Klassifizierung umfasst.

5. System (2) nach Anspruch 4, wobei der Algorithmus des Modells ein Prädiktionsansatz, insbesondere umfassend wenigstens ein lineares Filtermodell und/oder ein statistisches Modell, insbesondere umfassend wenigstens ein neuronales Netz und/oder ein Gauß'sches Mixturmodell, ist

6. System (2) nach einem der Ansprüche 1 bis 5, wobei das Überwachungsmodul (26) bei einer Spritzenpumpe Sensordaten erfasst und analysiert aus der Gruppe:
• Krallenwinkel, korreliert mit der verwendeten Spritze und ihrer Länge / ihrem Durchmesser als Funktion Zeit;
• Spritzenlänge / -durchmesser der verwendeten Spritze als Funktion der Zeit;
• Druckwerte während des Einlegens der Spritze und/oder während der Infusion,
und/oder wobei das Überwachungsmodul (26) bei einer Volumenpumpe/Peristaltikpumpe Sensordaten erfasst und analysiert aus der Gruppe
• Bewegung der Türpositionssensoren durch Überprüfung der Widerstandswerte über die Zeit und Korrelation der Werte mit den Motorschritten;
• Farbdetektion des eingelegten Schlauches, insbesondere anhand seiner Klemme, wobei der gemessene Farbwert mit der Bestätigung/Änderung durch den Benutzer ausgewertet wird;
• Erfassung von Drucksensorwerten über die Zeit, um zu überprüfen, ob sich im Laufe der Lebensdauer Abweichungen/Änderungen ergeben,
und/oder wobei das Überwachungsmodul (26) Sensordaten bei einer Volumenpumpe/Peristaltikpumpe mit einem Display oder Spritzenpumpe mit einem Display erfasst und analysiert aus der Gruppe
• Berührungssensorwerte an definierten Positionen auf dem Display, insbesondere auf der Schaltfläche "Start der Infusion" oder in Eingabefeldern als Funktion der Zeit;
• Austausch von Batterieinformationen durch ein Gerätemodell, insbesondere der Ladezustand als Funktion der Zeit, die Kapazität des Akkus, die Anzahl der Ladezyklen.

7. System (2) nach einem der vorherigen Ansprüche, umfassend eine Transmissionseinrichtung (62) zur Übertragung von Informationen von dem Überwachungsmodul (26) an die Infusionspumpe (6), und/oder wobei das Überwachungsmodul (26) ausgebildet ist, zu überwachen, ob die Sensordaten wenigstens eines Sensors (10, 14, 18) in einem vorgegebenen und/oder gelernten Nominalbereich liegen, und mit Hilfe der Transmissionseinrichtung (62) an die Infusionspumpe (6) Instruktionen zu senden und/oder ein Wartungsprogramm der Infusionspumpe (6) zu initiieren.

8. System (2) nach Anspruch 7, wobei die Instruktionen und/oder Schritte des Wartungsprogramm auf einem Display der Infusionspumpe (6) angezeigt werden.

9. System (2) nach einem der vorherigen Ansprüche, weiterhin umfassend einen Gesundheitsdaten-Server (38), welcher zum Empfang von Daten von dem Überwachungsmodul (26) und/oder zur Übertragung von Daten an das Überwachungsmodul (26) ausgebildet ist.

10. System (2) nach einem der vorherigen Ansprüche, wobei der Überwachungs-Server umfassend ein Trendanalysemodul (28), welches dazu ausgebildet ist, aufgrund übermittelter Sensordaten eine Trendanalyse durchzuführen, und/oder wobei das Trendanalysemodul (28) aufgrund der Trendanalyse eine Lebenszeiteinschätzung der Infusionspumpe (6) durchführt.

11. Verfahren zur Überwachung einer Infusionspumpe (6), umfassend die Verfahrensschritte
• Erfassen von Sensordaten einer Infusionspumpe (6);
• Eingeben der Sensordaten in ein mathematisches Modell;
• Klassifizieren des Zustandes der Infusionspumpe (6) durch das mathematische Modell.

12. Verfahren nach Anspruch 11, umfassend einen Anpassungs- und/oder Trainingsschritt für das mathematische Modell, in welchem mit Hilfe eines Trainingsdatensatzes das Modell angepasst und/oder trainiert wird, und/oder wobei

13. Verfahren nach Anspruch 11 oder 12, wobei der Trainingsdatensatz Daten wenigstens eines Sensors (10, 14, 18) und eine zugeordnete Klassifizierung umfasst.

14. Verfahren nach einem der Ansprüche 11 bis 13, umfassend einen Verfahrensschritt des Überwachens der Sensordaten wenigstens eines Sensors (10, 14, 18), wobei auf einem Display (66) der Infusionspumpe (6) Wartungsinstruktionen angezeigt werden, sofern die Sensordaten einen vorgegebenen und/oder gelernten Nominalbereich verlassen.

15. Computerprogramm-Produkt umfassend Instruktionen, welche, wenn sie auf einem Computer ausgeführt werden, ein Verfahren nach einem der Ansprüche 11-14 durchführen.
